# EUROPEAN PATENT SPECIFICATION

(11) **EP 2 194 957 B1**
(45) Date of publication and mention of the grant of the patent: **14.06.2017**
(21) Application number: 08839998.5
(22) Date of filing: 20.10.2008
(51) Int. Cl.: A61K 8/19, A61K 8/20, A61K 8/36, A61K 8/44, A61K 8/49, A61Q 5/06, A61Q 5/08, A61Q 5/10

(54) **COMPOSITION COMPRISING AT LEAST ONE AMMONIUM SALT, AQUEOUS AMMONIA AND AT LEAST ONE AMINO ACID**
ZUSAMMENSETZUNG AUS MINDESTENS EINEM AMMONIAKSALZ, WÄSSRIGEM AMMONIAK UND MINDESTENS EINER AMINOSÄURE
COMPOSITION COMPRENANT AU MOINS UN SEL D'AMMONIUM, DE L'AMMONIAC AQUEUX ET AU MOINS UN ACIDE AMINÉ

(30) Priority: 19.10.2007 FR 0758443
(43) Date of publication of application: 16.06.2010
(73) Proprietor: L'Oréal, 75008 Paris (FR)
(72) Inventor: LEGRAND, Frédéric, Westfield NJ 07090 (US); ASCIONE, Jean-Marc, F-75003 Paris (FR)
(74) Representative: Dossmann, Gérard
(86) International application number: PCT/EP2008/064112
(87) International publication number: WO 2009/050295

(56) References cited:
- EP-A- 1 484 048
- DATABASE WPI Week 200470 Thomson Scientific, London, GB; AN 2004-711531 XP002487584 -& JP 2004 262886 A (KANEBO LTD) 24 September 2004 (2004-09-24) cited in the application

## Description

The present invention relates to a composition for the treatment of keratin fibres, in particular human keratin fibres such as hair. The present invention also relates to methods for dyeing, bleaching and lightening keratin fibres using this composition and a device containing it.

It is known to use, for the treatment of hair, oxidizing compositions, in particular for dyeing human keratin fibres and in particular hair with dyeing compositions containing oxidation dye precursors, generally called oxidation bases. These oxidation bases are colourless or weakly coloured compounds which, when combined with oxidizing products, give rise, through a process of oxidative condensation, to coloured compounds.

The method of oxidation dyeing consists in applying to the keratin fibres oxidation bases or a mixture of oxidation bases and couplers with an oxidizing agent, such as hydrogen peroxide, which is added at the time of use.

Generally, this method is carried out at an alkaline pH, in particular in the presence of aqueous ammonia, and makes it possible to obtain dyeing and simultaneously lightening of the fibre which results in practice in the possibility of obtaining a final coloration which is lighter than the original colour. In addition, the lightening of the fibre has the advantageous effect of producing a single colour in the case of depigmented hair and, in the case of naturally pigmented hair, of enhancing the colour, that is to say of making it more visible.

It is also known to dye human keratin fibres by a so-called semipermanent coloration or direct coloration, which involves dyes capable of providing the natural coloration of the hair with a more or less marked modification, even without oxidizing agent.

These direct dyes may also be used in combination with oxidizing agents in the case where it is desired to obtain a lighter coloration than the original colour of the fibres. Thus, these direct dyes may be used in lightening direct dye compositions based on hydrogen peroxide and aqueous ammonia or in oxidation dye compositions in combination with oxidation bases and/or couplers.

Moreover, when a person wishes to bleach the hair, it is also known to perform bleaching using lightening products based on aqueous ammonia and hydrogen peroxide.

Thus, it is therefore customary to have recourse to alkaline oxidizing compositions based on hydrogen peroxide and aqueous ammonia for dyeing and/or bleaching human keratin fibres, and in particular hair.

However, even if these conditions of implementation prove to be effective, they can cause some inconvenience at the time of their use.

In particular, when these compositions are applied to the hair, there is in general a release of aqueous ammonia which can cause a suffocating and irritant odour for the eyes, for the respiratory tracts and the mucous membranes.

Furthermore, aqueous ammonia can cause, in particular in people having a sensitive scalp, reactions of discomfort such as redness, itching or tingling.

Finally, aqueous ammonia, in combination with an oxidizing agent, can also contribute to damaging the keratin fibres. Indeed, in the long term, it is observed that the fibres are degraded to a greater or lesser degree and have a tendency to become rough, dull, brittle and difficult to style.

Thus, to overcome all the disadvantages described above, numerous alternatives have already been proposed in order to significantly reduce the content of aqueous ammonia in compositions intended for dyeing and/or bleaching fibres.

To this effect, it has been proposed to apply to the hair dyeing and/or bleaching compositions comprising a nonvolatile organic amine, such as monoethanolamine. While such compositions have the advantage of not releasing ammonia during their use, these compositions most often cause reactions of discomfort, in particular irritations in people having a sensitive scalp. Furthermore, for equivalent lightening performance, monoethanolamine damages the hair more than aqueous ammonia.

Other compositions combining aqueous ammonia with a water-soluble salt of ammonium have also been envisaged. Such compositions are in particular described in patent application EP148466.

However, these compositions do not make it possible to satisfactorily reduce the unpleasant odours caused by the release of ammonia and the lightening performance of this type of compositions remains limited compared to compositions based on aqueous ammonia.

Likewise, compositions containing compounds such as ammonium or alkali metal or alkaline-earth metal carbonates, carbamates or hydrogen carbonates have also been proposed.

Even if these compositions make it possible to significantly reduce the aqueous ammonia contents, the lightening performance of this type of compositions is still less than that of aqueous ammonia compositions. Furthermore, these compositions continue to greatly damage the keratin fibres.

Alternatively, compositions based on neutral or basic amino acids have also been envisaged in order to completely or partially replace aqueous ammonia.

Thus, patent EP0840593 describes aqueous ammonia-free compositions comprising in particular, as alkaline agent, a mixture based on a compound chosen from amino acids and oligopeptides having an amino group and a -COOH or -SO₃H group and on a compound chosen from the group formed of monoethanolamine, monoisopropanolamine, 2-amino-2-methylpropanol, 2-amino-2-methyl-1,3-propanediol, 2-amino-2-ethyl-1,3-propanediol and 2-amino-2-methylbutanol.

Likewise, patent applications JP2004262885 and JP2004262886 describe aqueous ammonia-free compositions respectively based on neutral or basic amino acids, a nonvolatile amine and an organic acid ion of ammonium.

Finally, patent US5131912 describes compositions based on neutral or basic amino acids as alkaline agents, such as ammonium or alkali metal or alkaline-earth metal carbonates or hydrogen carbonates. The mixture before using these alkaline compositions with an oxidizing composition of hydrogen peroxide has a pH of between 6.5 and 7.9.

While such compositions have the advantage of not releasing ammonia during their use, these compositions still do not make it possible to match the level of performance of the aqueous ammonia-based compositions in terms of lightening. Furthermore, these compositions can cause irritation of the scalp.

Thus, a real need therefore exists to reduce the aqueous ammonia contents in compositions intended in particular for dyeing, in order to reduce the unpleasant odours accompanying it, the irritation of the scalp and the damage to the keratin fibres while preserving good dyeing properties.

The subject of the present invention is therefore in particular a composition comprising, in a cosmetically acceptable medium:
(a) one or more ammonium salts of an inorganic acid or an organic acid having from 1 to 7 carbon atoms,
(b) aqueous ammonia, and
(c) one or more basic amino acids; wherein the basic amino acid(s) are present in an amount ranging from 0.5 to 10% by weight, relative to the total weight of the cosmetic composition,
   and
(d) one or more oxidation dye precursors. Thus, the composition according to the invention has the advantage of minimizing the disadvantages due to a release of ammonia.

The composition according to the invention also makes it possible to reduce the discomfort which can be felt at the time of application of the the said composition to the keratin fibres in the region of the scalp.

Furthermore, the composition makes it possible to reduce the damage to the fibre compared with conventional dyeing and/or bleaching compositions containing aqueous ammonia as principal alkaline agent. The dyeing composition obtained has the additional advantage of preserving good dyeing properties which make it possible to obtain intense colorations which are chromatic, little selective and quite resistant to various attacks to which the hair may be subjected.

The present invention also relates to methods for dyeing keratin fibres and multicompartment dyeing devices or "kits".

Other subjects, characteristics, aspects and advantages of the invention will emerge even more clearly on reading the description and examples which follow.

In the text which follows, unless otherwise stated, the limits of the intervals indicated are included in the invention.

The ammonium salts used in the oxidizing composition according to the invention are ammonium salts of an inorganic acid or of an organic acid having from 1 to 7 carbon atoms.

By way of examples of ammonium salts of inorganic acids or of organic acids having from 1 to 7 carbon atoms used in the oxidizing composition according to the invention, there may be mentioned in particular ammonium carbonate, ammonium carbamate, ammonium hydrogen carbonate, ammonium sulphates, ammonium glycerophosphates, ammonium chloride, ammonium nitrate, ammonium acetate, ammonium thiolactate, ammonium fumarate and ammonium salts of hydroxy acids such as ammonium gluconate, ammonium citrate, tartrate, lactate, malate or ammonium salts of amino acids such as aspartate, arginate, glycocholate.

Preferably, the ammonium salt(s) of inorganic acids or of organic acids having from 1 to 7 carbon atoms are chosen from ammonium carbonate, ammonium chloride, ammonium acetate, ammonium hydrogen carbonate, ammonium lactate and ammonium thiolactate. According to a particular embodiment, the ammonium salt is chosen from ammonium carbonate, ammonium chloride and ammonium acetate.

The ammonium salt(s) of inorganic or organic acids having from 1 to 7 carbon atoms are present in the composition according to the invention in an amount ranging from 0.1 to 15% by weight, preferably ranging from 0.5 to 10% by weight, relative to the total weight of the cosmetic composition.

The aqueous ammonia may be present in the oxidizing composition according to the invention in an amount preferably less than 20% by weight, preferably ranging from 0.5 to 15% by weight, relative to the total weight of the cosmetic composition, this being expressed using an aqueous solution containing the equivalent of 20% ammonia.

The expression "amino acid" is understood to mean, for the purposes of the present invention, a compound which is not obtained following the polycondensation of identical or different amino acids.

The amino acids used in the composition acording to the invention contain at least one amine functional group and at least one acid functional group. The acid functional group(s) may be carboxylic, sulphonic, phosphonic or phosphoric, preferably carboxylic, acid functional groups.

Preferably, the amino acids used in the present invention are α-amino acids, that is to say that they contain an amine functional group and a group R located at the alpha position with respect to the acid functional group. They may be represented by the following formula: 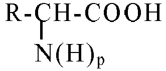 in which:
when p=2, R represents a hydrogen atom, an aliphatic group containing or not containing a heterocyclic portion, or an aromatic group,
   or
when p=1, R may form with the nitrogen atom of -N(H)ₚ a heterocycle. This heterocycle is preferably a 5-membered saturated ring, optionally substituted with one or more C₁₋₄ alkyl or hydroxyl groups.

Preferably, the aliphatic group is a linear or branched C₁₋₄ alkyl group; a linear or branched C₁₋₄ hydroxyalkyl group; a linear or branched C₁₋₄ aminoalkyl group; a linear or branched (C₁₋₄ alkyl)thio(C₁₋₄ alkyl) group; a linear or branched C₂₋₄ carboxyalkyl group; a linear or branched ureidoalkyl group, a linear or branched guanidinoalkyl group, a linear or branched imidazoloalkyl group, a linear or branched indoylalkyl group, the alkyl portions of the latter four groups containing one to four carbon atoms.

Preferably, the aromatic group is a C₆ aryl or C₇₋₁₀ aralkyl group, the aromatic nucleus being optionally substituted with one or more C₁₋₄ alkyl or hydroxyl groups.

More particularly, the basic amino acids are chosen from arginine, histidine and lysine. According to a particular embodiment, the basic amino acid is chosen from arginine and lysine.

The compositions used in accordance with the invention have a concentration of amino acid(s) between 1 and 10% by weight relative to the total weight of the composition.

The composition may comprise one or more oxidizing agents chosen, for example, from hydrogen peroxide, urea peroxide, alkali metal bromates, persalts such as perborates and persulphates, enzymes such as peroxidases and oxidoreductases with two or four electrons. The use of hydrogen peroxide is particularly preferred.

The content of oxidizing agent in the composition may be between 0.1 and 10% by weight of the composition, preferably between 0.5 and 6% by weight of the composition.

Preferably, the pH of the composition after mixing with the oxidizing agent is between 5 and 10.5, preferably between 6 and 10.

Preferably, the composition according to the invention comprises one or more surfactants chosen from anionic, nonionic, cationic, amphoteric or zwitterionic surfactants.

By way of example of anionic surfactants which can be used, alone or as mixtures, in the context of the present invention there may be mentioned in particular (nonlimiting list) the salts (in particular alkali metal, especially sodium, salts, ammonium salts, amine salts, amino alcohol salts or magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamidesulphonates, alkyl aryl sulphonates, α-olefinsulphonates, paraffinsulphonates; (C₆-C₂₄)alkyl sulphosuccinates, (C₆-C₂₄)alkyl ether sulphosuccinates, (C₆-C₂₄)alkylamide sulphosuccinates; (C₆-C₂₄)alkyl sulphoacetates; (C₆-C₂₄)acyl sarcosinates and (C₆-C₂₄)acyl glutamates. It is also possible to use (C₆-C₂₄)alkyl polyglycoside carboxylic esters such as alkyl glucoside citrates, alkyl polyglycoside tartrate and alkyl polyglycoside sulphosuccinates, alkyl sulphosuccinamates; acyl isethionates and N-acyltaurates, the alkyl or acyl radical of all these various compounds preferably comprising from 12 to 20 carbon atoms, and the aryl radical preferably denoting a phenyl or benzyl group. Among the anionic surfactants which can still be used, there may also be mentioned the salts of fatty acids such as the salts of oleic, ricinoleic, palmitic and stearic acids, the acids of copra oil or of hydrogenated copra oil, and in particular preferably the sodium, calcium or magnesium salts of stearic acid; the acyllactylates whose acyl radical comprises 8 to 20 carbon atoms. It is also possible to use the alkyl D-galactoside uronic acids and their salts, the polyoxyalkylenated (C₆-C₂₄)alkyl ether carboxylic acids, the polyoxyalkylenated (C₆-C₂₄)alkylaryl ether carboxylic acids, the polyoxyalkylenated (C₆-C₂₄)alkylamido ether carboxylic acids and their salts, in particular those comprising from 2 to 50 alkylene, in particular ethylene, oxide groups, and mixtures thereo f.

The nonionic surfactants which can be used in the compositions according to the invention are compounds which are well known per se (in this respect see especially the "Handbook of Surfactants" by M.R. Porter, published by Blackie & Son (Glasgow and London), 1991, pp. 116-178). They can thus be chosen especially from (nonlimiting list) alcohols, alpha-diols or polyethoxylated or polypropoxylated alkylphenols which have a fatty chain containing, for example, 8 to 18 carbon atoms, it being possible for the number of ethylene oxide or propylene oxide groups to range especially from 2 to 50. The copolymers of ethylene oxide and propylene oxide and the condensates of ethylene oxide and propylene oxide with fatty alcohols may also be mentioned; the polyethoxylated fatty amides preferably containing from 2 to 30 mol of ethylene oxide, the polyglycerolated fatty amides containing on average 1 to 5 glycerol groups and in particular 1.5 to 4; the polyethoxylated fatty amines preferably having 2 to 30 moles of ethylene oxide; the oxyethylenated fatty acid esters of sorbitan containing from 2 to 30 mol of ethylene oxide; the fatty acid esters of sucrose, the fatty acid esters of polyethylene glycol, alkylpolyglycosides, the N-alkylglucamine derivatives, amine oxides such as the oxides of (C₁₀-C₁₄)alkylamines or the N-acylaminopropylmorpholine oxides.

The amphoteric or zwitterionic surfactants which can be used in the compositions according to the present invention may be especially (nonlimiting list) derivatives of aliphatic secondary or tertiary amines in which the aliphatic radical is a linear or branched chain containing 8 to 18 carbon atoms and containing at least one water-solubilizing anionic group (for example carboxylate, sulphonate, sulphate, phosphate or phosphonate); (C₈-C₂₀)alkylbetaines, sulphobetaines, (C₈-C₂₀)alkylamido(C₁-C₆)alkylbetaines or (C₈-C₂₀)alkylamido(C₁-C₆)alkylsulphobetaines may further be mentioned.

Among the amine derivatives, there may be mentioned the products sold under the name MIRANOL, as described in patents US-2 528 378 and US-2 781 354 and classified in the CTFA dictionary, 3rd edition, 1982, under the names Amphocarboxyglycinates and Amphocarboxypropionates having the respective structures:

R₂-CONHCH₂CH₂-N(R₃)(R₄)(CH₂COO-) (I)

in which: R₂ denotes an alkyl radical of an acid R₂-COOH present in hydrolysed copra oil, a heptyl, nonyl or undecyl radical, R₃ denotes a beta-hydroxyethyl group and R₄ a carboxymethyl group;
and

R₂'-CONHCH₂CH₂-N(B)(C) (I')

in which:
B represents -CH₂CH₂OX', C represents -(CH₂)_{z}-Y', with z = 1 or 2,
X' denotes the -CH₂CH₂-COOH group or a hydrogen atom,
Y' denotes -COOH or the radical -CH₂-CHOH-SO₃H,
R₂' denotes an alkyl radical of an acid R₉ -COOH present in copra oil or in hydrolysed linseed oil, an alkyl radical, especially C₇, C₉, C₁₁ or C₁₃, a C₁₇ alkyl radical and its iso form or an unsaturated C₁₇ radical.

These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylampho-dipropionate, Disodium Capryloamphodipropionate, Lauroamphodipropionic acid, Cocoamphodipropionic acid.

By way of example, there may be mentioned the cocoamphodiacetate marketed under the trade name MIRANOL® C2M concentrated by the company RHODIA CHIMIE.

Among the cationic surfactants which can be used in the composition according to the invention, there may be mentioned in particular (nonlimiting list): the salts of optionally polyoxyalkylenated primary, secondary or tertiary amines; quaternary ammonium salts such as tetraalkylammonium, alkylamidoalkyltrialkylammonium, trialkylbenzylammonium, trialkylhydroxyalkylammonium or alkylpyridinium chlorides or bromides; imidazoline derivatives or amine oxides of a cationic nature.

The composition according to the invention may additionally contain at least one fatty substance. The fatty substances which can be used in the present invention are chosen in particular from vegetable oils, animal oils, mineral oils, natural or synthetic oils, fatty alcohols and mixtures thereof.

The composition according to the invention may also contain one or more thickeners also called "rheology-adjusting agents".

As rheology-adjusting agents, nonassociative gelling agents may be used; among these are polymers or copolymers of unsaturated organic carboxylic acids or of unsaturated esters, polysaccharide derivatives, gums, colloidal silicates, polyethylene glycols, polyvinylpyrrolidones, hydrophilic silica gels.

The rheology-adjusting agents may also be chosen from fatty acid amides (diethanol- or monoethanolamide of copra, monoethanolamide of oxyethylenated alkyl ether carboxylic acid), cellulosic thickeners (hydroxyethylcellulose, hydroxypropylcellulose, carboxymethylcellulose), guar gum and its derivatives (hydroxypropylguar), gums of microbial origin (xanthan gum, scleroglucan gum), crosslinked homopolymers or copolymers of acrylic acid or of acrylamidopropanesulphonic acid and the associative thickening polymers.

These associative polymers are water-soluble polymers which are capable, in an aqueous medium, of reversibly combining with each other or with other molecules.

Their chemical structure comprises hydrophilic regions, and hydrophobic regions which are characterized by at least one fatty chain.

The associative polymers may be of the anionic, cationic, amphoteric or nonionic type.

Anionic associative polymers are for example described and prepared, according to an emulsion polymerization method, in patent EP-0 216 479.

Among these anionic associative polymers, the polymers formed from 20 to 60% by weight of acrylic acid and/or of methacrylic acid, from 5 to 60% by weight of lower alkyl (meth)acrylates, from 2 to 50% by weight of allyl ether containing a fatty chain, and from 0 to 1% by weight of a crosslinking agent which is a well-known copolymerizable polyethylenic unsaturated monomer such as diallyl phthalate, allyl (meth)acrylate, divinylbenzene, (poly)ethylene glycol dimethacrylate and methylenebisacrylamide, are particularly preferred according to the invention.

Among the latter, the crosslinked terpolymers of methacrylic acid, ethyl acrylate, polyethylene glycol (10 EO) stearyl alcohol ether (Steareth 10), in particular those sold by the company ALLIED COLLOIDS under the names SALCARE SC80® and SALCARE SC90® which are aqueous emulsions containing 30% of a crosslinked terpolymer of methacrylic acid, of ethyl acrylate and of steareth-10-allyl ether (40/50/10) are most particularly preferred.

Mention may also be made of (C₁₀-C₃₀)alkyl esters of unsaturated carboxylic acids in accordance with the invention which comprise for example lauryl acrylate, stearyl acrylate, decyl acrylate, isodecyl acrylate, dodecyl acrylate, and the corresponding methacrylates, lauryl methacrylate, stearyl methacrylate, decyl methacrylate, isodecyl methacrylate and dodecyl methacrylate.

Anionic polymers of this type are for example described and prepared according to patents US-3 915 921 and 4 509 949.

Mention may also be made of the products sold by the company GOODRICH under the trade names PEMULEN TR1®, PEMULEN TR2®, CARBOPOL 1382®, and still more preferably PEMULEN TR1®, and the product sold by the company S.E.P.P.I.C. under the name COATEX SX®.

By way of example of this type of compound, there may be mentioned ACULYN 22® sold by the company ROHM and HAAS, which is an oxyalkylenated stearyl methacrylate/ethyl acrylate/methacrylic acid terpolymer.

Among the associative polymers of the cationic type, there may be mentioned:
- (I) the cationic associative polyurethanes, the family of which has been described by the applicant in French patent application No. 0009609.
   The number-average molecular mass of the cationic associative polyurethanes is preferably between 400 and 500 000, and in particular between 1000 and 400 000, and ideally between 1000 and 300 000.
- (II) the quaternized cellulose derivatives and the polyacrylates with noncyclic amine-containing side groups.

The quaternized cellulose derivatives are in particular,
- the quaternized celluloses modified by groups comprising at least one fatty chain, such as the alkyl, arylalkyl or alkylaryl groups comprising at least 8 carbon atoms, or mixtures thereof,
- the quaternized hydroxyethylcelluloses modified by groups comprising at least one fatty chain, such as the alkyl, arylalkyl or alkylaryl groups comprising at least 8 carbon atoms, or mixtures thereof.

The alkyl radicals carried by the above quaternized celluloses or hydroxyethylcelluloses preferably comprise from 8 to 30 carbon atoms. The aryl radicals preferably denote phenyl, benzyl, naphthyl or anthryl groups.

There may be mentioned, as examples of quaternized alkylhydroxyethylcelluloses containing C₈-C₃₀ fatty chains, the products QUATRISOFT LM 200®, QUATRISOFT LM-X 529-18-A®, QUATRISOFT LM-X 529-18B® (C₁₂ alkyl) and QUATRISOFT LM-X 529-8® (C₁₈ alkyl) marketed by the company AMERCHOL and the products CRODACEL QM®, CRODACEL QL® (C₁₂ alkyl) and CRODACEL QS® (C₁₈ alkyl) marketed by the company CRODA.

The amphoteric associative polymers are preferably chosen from those containing at least one noncyclic cationic unit. Still more particularly, the ones that are preferred are those prepared from or comprising 1 to 20 mol% of monomer containing a fatty chain, and preferably 1.5 to 15 mol% and still more particularly 1.5 to 6 mol%, relative to the total number of moles of monomers.

Amphoteric associative polymers are for example described and prepared in patent application WO9844012.

Among the amphoteric associative polymers according to the invention, the acrylic acid/(meth)acrylamidopropyltrimethylammonium chloride/stearyl methacrylate terpolymers are preferred.

The associative polymers of the nonionic type which can be used according to the invention are preferably chosen from:
- (1) celluloses modified by groups comprising at least one fatty chain;
   there may be mentioned by way of example:
   - the hydroxyethylcelluloses modified by groups comprising at least one fatty chain such as alkyl, arylalkyl or alkylaryl groups, or mixtures thereof, and in which the alkyl groups are preferably C₈-C₂₂, such as the product NATROSOL PLUS GRADE 330 CS® (C₁₆ alkyls) sold by the company AQUALON, or the product BERMOCOLL EHM 100® sold by the company BEROL NOBEL,
   - those modified by polyalkylene glycol ether of alkylphenol groups, such as the product AMERCELL POLYMER HM-1500® (polyethylene glycol (15) ether of nonylphenol) sold by the company AMERCHOL.
- (2) hydroxypropylguars modified by groups comprising at least one fatty chain such as the product ESAFLOR HM 22® (C₂₂ alkyl chain) sold by the company LAMBERTI, the products RE210-18® (C₁₄ alkyl chain) and RE205-1® (C₂₀ alkyl chain) sold by the company RHONE POULENC.
- (3) copolymers of vinylpyrrolidone and of hydrophobic monomers having a fatty chain, of which there may be mentioned by way of example:
   - the products ANTARON V216® or GANEX V216® (vinylpyrrolidone/hexadecene copolymer) sold by the company I.S.P.
   - the products ANTARON V220® or GANEX V220® (vinylpyrrolidone/eicosene copolymer) sold by the company I.S.P.
- (4) copolymers of C₁-C₆ alkyl methacrylates or acrylates and of amphiphilic monomers comprising at least one fatty chain such as for example the oxyethylenated stearyl acrylate/methyl acrylate copolymer sold by the company GOLDSCHMIDT under the name ANTIL 208®.
- (5) copolymers of hydrophilic methacrylates or acrylates and of hydrophobic monomers comprising at least one fatty chain such as for example the polyethylene glycol methacrylate/lauryl methacrylate copolymer.
- (6) polyether-polyurethanes comprising in their chain both hydrophilic sequences which are most often of a polyoxyethylenated nature and hydrophobic sequences which may be aliphatic chains alone and/or cycloaliphatic and/or aromatic chains.
- (7) polymers containing an aminoplast ether backbone possessing at least one fatty chain, such as the compounds PURE THIX® provided by the company SUD-CHEMIE.

By way of examples of nonionic polyether-polyurethanes containing a fatty chain which can be used in the invention, it is also possible to use Rhéolate 205® containing a urea functional group sold by the company RHEOX or the Rhéolates® 208, 204 or 212, as well as Acrysol RM 184®.

There may also be mentioned the product ELFACOS T210® containing a C₁₂-₁₄ alkyl chain and the product ELFACOS T212® containing a C₁₈ alkyl chain from AKZO.

The product DW 1206B® from RHOM & HAAS containing a C₂₀ alkyl chain and with a urethane bond, sold at 20% dry matter content in water, may also be used.

It is also possible to use solutions or dispersions of these polymers in particular in water or in an aqueous alcoholic medium. By way of example of such polymers, there may be mentioned Rhéolate® 255, Rhéolate® 278 and Rhéolate® 244 sold by the company RHEOX. It is also possible to use the product DW 1206F and DW 1206J provided by the company ROHM & HAAS.

There may also be mentioned the polyether-polyurethanes sold in particular by the company ROHM & HAAS under the names Aculyn 46® and Aculyn 44® [ACULYN 46® is a polycondensate of polyethylene glycol containing 150 or 180 mol of ethylene oxide, stearyl alcohol and methylenebis(4-cyclohexyl isocyanate) (SMDI), at 15% by weight in a matrix of maltodextrin (4%) and water (81%); ACULYN 44® is a polycondensate of polyethylene glycol containing 150 or 180 mol of ethylene oxide, decyl alcohol and methylenebis(4-cyclohexyl isocyanate) (SMDI), at 35% by weight in a mixture of propylene glycol (39%) and water (26%)].

Preferably, the thickening polymers are chosen from the associative polymers of the cationic type such as quaternized celluloses modified by groups containing at least one fatty chain, quaternized hydroxyethylcelluloses modified by groups containing at least one fatty chain and associative polymers of the nonionic type such as celluloses modified by groups containing at least one fatty chain and hydroxyethylcelluloses modified by groups containing at least one fatty chain.

The quantity of thickening agents present in the composition according to the invention is between 0.01 and 10%, and preferably between 0.1 and 5% by weight relative to the total weight of the composition.

The composition according to the invention may also contain one or more anhydrous cationic or amphoteric conditioning polymers such as, for example, those described in French patents Nos. 2788974 and 2788976 and as described below.

For the purposes of the preent invention, the expression "cationic polymer" denotes any polymer containing cationic groups and/or groups that are ionizable to cationic groups.

The expression "conditioning polymer" is understood to mean, for the purposes of the present invention, a polymer capable of improving the cosmetic properties of the keratin fibres, such as disentanglement, feel, smoothness, shine and volume.

The cationic polymers which can be used in accordance with the present invention may be chosen from all those already known per se as improving the cosmetic properties of hair, namely in particular those described in patent application EP-A-337 354 and in French patents FR-2 270 846, 2 383 660, 2 598 611, 2 470 596 and 2 519 863.

The preferred cationic polymers are chosen from those which contain units comprising primary, secondary, tertiary and/or quaternary amine groups which may either be part of the main polymer chain, or which may be carried by a side substituent directly linked to the latter.

The cationic polymers used generally have a number-average molecular mass between 500 and 5.10⁶ approximately, and preferably between 10³ and 3.10⁶ approximately.

Among the cationic polymers, there may be mentioned more particularly polymers of the polyamine, polyamino amide and poly(quaternary ammonium) type.

They are known products. They are described in particular in French patents No. 2 505 348 or 2 542 997. Among the the said polymers, there may be mentioned:
(1) The homopolymers or copolymers derived from acrylic or methacrylic esters or amides and comprising at least one of the units of the following formulae (II), (III), (IV) or (V): 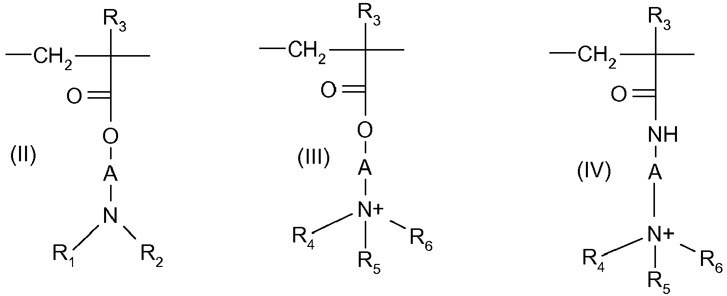 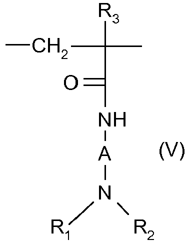 in which:
   R₃, which are identical or different, denote a hydrogen atom or a CH₃ radical;
   A, which are identical or different, represent a linear or branched alkyl group of 1 to 6 carbon atoms, preferably 2 or 3 carbon atoms or a hydroxyalkyl group of 1 to 4 carbon atoms;
   R₄, R₅, R₆, which are identical or different, represent an alkyl group having from 1 to 18 carbon atoms or a benzyl radical and preferably an alkyl group having from 1 to 6 carbon atoms;
   R₁ and R₂, which are identical or different, represent hydrogen or an alkyl group having from 1 to 6 carbon atoms and preferably methyl or ethyl;
   X⁻ denotes an anion derived from an inorganic or organic acid such as a methosulphate anion or a halide such as chloride or bromide.

   The polymers of the family (1) may contain, in addition, one or more units derived from comonomers which may be chosen from the family of acrylamides, methacrylamides, diacetone acrylamides, acrylamides and methacrylamides substituted on the nitrogen with lower (C₁-C₄)alkyls, acrylic or methacrylic acids or esters thereof, vinyllactams such as vinylpyrrolidone or vinylcaprolactam, vinyl esters.
   Thus, among these polymers of the family (1), there may be mentioned:
   - the copolymers of acrylamide and dimethylaminoethyl methacrylate quaternized with dimethyl sulphate or with a dimethyl halide;
   - the copolymers of acrylamide and methacryloyloxyethyltrimethylammonium chloride described, for example, in Patent Application EP-A-080976;
   - the copolymer of acrylamide and methacryloyloxyethyltrimethylammonium methosulphate;
   - the vinylpyrrolidone/dialkylaminoalkyl acrylate or methacrylate copolymers, quaternized or otherwise. These polymers are described in detail in French patents 2 077 143 and 2 393 573;
   - the dimethylaminoethyl methacrylate/vinylcaprolactam/vinylpyrrolidone terpolymers;
   - the vinylpyrrolidone/methacrylamidopropyldimethylamine copolymers;
   - the quaternized vinylpyrrolidone/dimethyl aminopropyl methacrylamide copolymers;
   - the crosslinked polymers of methacryloyloxy(C₁-C₄ alkyl)tri(C₁-C₄)alkylammonium salts such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with a compound containing olefinic unsaturation, in particular methylenebisacrylamide. More particularly, it is possible to employ a crosslinked acrylamide/methacryloyloxyethyltrimethylammonium chloride copolymer (20/80 by weight) in the form of a dispersion containing 50% by weight of the the said copolymer in mineral oil. This dispersion is marketed under the name of "SALCARE® SC 92" by the company ALLIED COLLOIDS. It is also possible to employ a crosslinked methacryloyloxyethyltrimethylammonium chloride homopolymer containing approximately 50% by weight of the homopolymer in mineral oil or in a liquid ester. These dispersions are marketed under the names of "SALCARE® SC 95" and "SALCARE® SC 96" by the company ALLIED COLLOIDS.
(2) The cellulose ether derivatives comprising quaternary ammonium groups, described in French Patent 1 492 597. These polymers are also defined in the CTFA dictionary as hydroxyethyl cellulose quaternary ammoniums which have reacted with an epoxide substituted by a trimethylammonium group.
(3) Cellulose copolymers or cellulose derivatives grafted with a quaternary ammonium water-soluble monomer, and described especially in US Patent 4 131 576, such as hydroxyalkyl celluloses like hydroxymethyl-, hydroxyethyl- or hydroxypropyl celluloses grafted especially with a methacryloylethyltrimethylammonium, methacrylamidopropyltrimethylammonium or dimethyldiallylmmonium salt.
(4) The cationic polygalactomannans described more particularly in US Patents 3 589 578 and 4 031 307 such as guar gums containing cationic trialkylammonium groups. Guar gums modified with a 2,3-epoxypropyltrimethylammonium salt (e.g. chloride) are for example used.
(5) Polymers consisting of piperazinyl units and of alkylene or hydroxyalkylene divalent radicals with straight or branched chains, optionally interrupted by oxygen, sulphur or nitrogen atoms or by aromatic or heterocyclic rings, as well as the oxidation and/or quaternization products of these polymers. Such polymers are described especially in French patents 2 162 025 and 2 280 361.
(6) Water-soluble polyaminoamides prepared in particular by polycondensation of an acid compound with a polyamine; these polyaminoamides may be crosslinked with an epihalohydrin, a diepoxide, a dianhydride, an unsaturated dianhydride, a diunsaturated derivative, a bishalohydrin, a bisazetidinium, a bishaloacyldiamine, an alkylbishalide or else with an oligomer resulting from the reaction of a difunctional compound which is reactive towards a bishalohydrin, a bisazetidinium, a bishaloacyldiamine, an alkylbishalide, an epihalohydrin, a diepoxide or a diunsaturated derivative; the crosslinking agent being employed in proportions ranging from 0.025 to 0.35 mol per amine group of the polyaminoamide; these polyaminoamides may be alkylated or, if they include one or more tertiary amine functional groups, quaternized. Such polymers are described especially in French Patents 2 252 840 and 2 368 508.
(7) Polyaminoamide derivatives resulting from the condensation of polyalkylenepolyamines with polycarboxylic acids, followed by an alkylation with difunctional agents. There may be mentioned, for example, the adipic acid/dialkylaminohydroxy-alkyldialkylenetriamine polymers in which the alkyl radical contains from 1 to 4 carbon atoms and preferably denotes methyl, ethyl or propyl. Such polymers are described especially in French Patent 1 583 363.
   Among these derivatives there may be mentioned more particularly the adipic acid/dimethyl aminohydroxy-propyl/diethylenetriamine polymers.
(8) Polymers obtained by reaction of a polyalkylenepolyamine containing two primary amine groups and at least one secondary amine group with a dicarboxylic acid chosen from diglycolic acid and saturated aliphatic dicarboxylic acids containing from 3 to 8 carbon atoms. The molar ratio of the polyalkylenepolyamine to the dicarboxylic acid being between 0.8:1 and 1.4:1; the polyaminoamide resulting therefrom being made to react with epichlorohydrin in a molar ratio of epichlorohydrin relative to the secondary amine group of the polyaminoamide of between 0.5 : 1 and 1.8 : 1. Such polymers are described especially in American Patents 3 227 615 and 2 961 347.
(9) Cyclopolymers of alkyldiallylamine or of dialkyldiallylammonium, such as the homopolymers or copolymers comprising, as main constituent of the chain, units corresponding to the formulae (VI) or (VII): 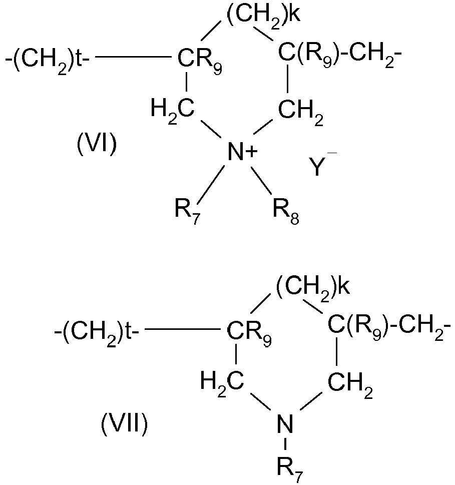 in which formulae k and t are equal to 0 or 1, the sum k + t being equal to 1; R₉ denotes a hydrogen atom or a methyl radical; R₇ and R₈, independently of each other, denote an alkyl group containing from 1 to 6 carbon atoms, a hydroxyalkyl group in which the alkyl group preferably has 1 to 5 carbon atoms, or a lower (C₁-C₄)amidoalkyl group or R₇ and R₈ may denote, jointly with the nitrogen atom to which they are attached, heterocyclic groups such as piperidinyl or morpholinyl; R₇ and R₈, independently of each other, preferably denote an alkyl group having 1 to 4 carbon atoms; Y⁻ is an anion such as bromide, chloride, acetate, borate, citrate, tartrate, bisulphate, bisulphite, sulphate or phosphate. These polymers are described especially in French Patent 2 080 759 and in its certificate of addition 2 190 406.
(10) The quaternary diammonium polymer containing repeat units corresponding to the formula: 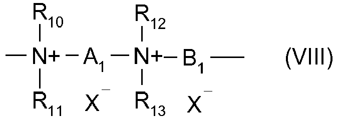
   formula (VIII) in which:
      R₁₀, R₁₁, R₁₂ and R₁₃, which are identical or different, represent aliphatic, alicyclic or arylaliphatic radicals containing from 1 to 20 carbon atoms or lower hydroxyalkyl aliphatic radicals, or else R₁₀, R₁₁, R₁₂ and R₁₃, together or separately, form, with the nitrogen atoms to which they are attached, heterocyclic rings optionally containing a second heteroatom other than nitrogen, or else R₁₀, R₁₁, R₁₂ and R₁₃ denote a linear or branched C₁-C₆ alkyl radical substituted by a nitrile, ester, acyl, amide or -CO-O-R₁₄-D or -CO-NH-R₁₄-D group where R₁₄ is an alkylene and D a quaternary ammonium group;
      A₁ and B₁ represent polymethylene groups containing from 2 to 20 carbon atoms which may be linear or branched, saturated or unsaturated and which may contain, bonded to or inserted into the main chain, one or more aromatic rings, or one or more oxygen or sulphur atoms or sulphoxide, sulphone, disulphide, amino, alkylamino, hydroxyl, quaternary ammonium, ureido, amide or ester groups, and
      X⁻ denotes an anion derived from an inorganic or organic acid;
      A1, R₁₀ and R₁₂, with the two nitrogen atoms to which they are attached, may form a piperazine ring; in addition if A₁ denotes a saturated or unsaturated, linear or branched alkylene or hydroxyalkylene radical, B₁ may also denote a group -(CH₂)n-CO-DOC-(CH₂)n- in which n is between 1 and 100 and preferably between 1 and 50, and D denotes:
         a) a glycol residue of formula: -O-Z-O-, where Z denotes a linear or branched hydrocarbon radical or a group corresponding to one of the following formulae:

            -(CH₂-CH₂-O)x-CH₂-CH₂-

            -[CH₂-CH(CH₃)-O]y-CH₂-CH(CH₃)-

            where x and y denote an integer from 1 to 4, representing a defined and unique degree of polymerization or any number from 1 to 4 representing a mean degree of polymerization;
         b) a disecondary diamine residue such as a piperazine derivative;
         c) a diprimary diamine residue of formula: -NH-Y-NH-, where Y denotes a linear or branched hydrocarbon radical or else the divalent radical

            -CH₂-CH₂-S-S-CH₂-CH₂-;
         d) a ureylene group of formula: -NH-CO-NH-.

      X⁻ is preferably an anion such as chloride or bromide.
      These polymers have a number-average molecular mass which is generally between 1000 and 100 000.
      Polymers of this type are described especially in French Patents 2 320 330, 2 270 846, 2 316 271, 2 336 434 and 2 413 907 and US Patents 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 and 4 027 020.
      It is possible to use more particularly the polymers which consist of repeat units corresponding to the following formula (IX): 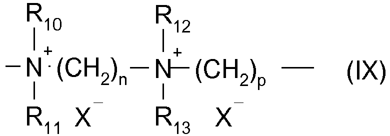 in which R₁₀, R₁₁, R₁₂ and R₁₃, which are identical or different, denote an alkyl or hydroxyalkyl radical having from 1 to 4 carbon atoms approximately, n and p are integers varying from 2 to 20 approximately and X⁻ is an anion derived from an inorganic or organic acid.
(11) The quaternary polyammonium polymers consisting of repeat units of formula (X): 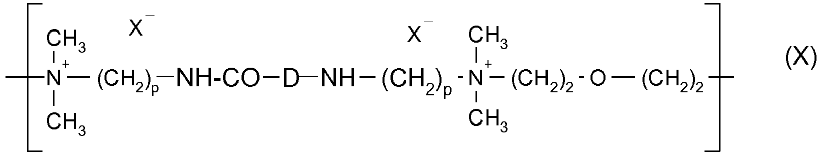 in which p denotes an integer varying from 1 to 6 approximately, D may be zero or may represent a group -(CH₂)ᵣ-CO- in which r denotes a number equal to 4 or to 7, X⁻ is an anion.
   Such polymers may be prepared according to the methods described in USA Patents No. 4 157 388, 4 702 906, 4 719 282. They are in particular described in patent application EP-A-122 324.
(12) Quaternary vinylpyrrolidone and vinylimidazole polymers.
(13) Polyamines such as the product referenced under the name of "POLYETHYLENE GLYCOL (15) TALLOW POLYAMINE" in the CTFA dictionary.

Other cationic polymers that may be employed within the scope of the invention are polyalkyleneimines, in particular polyethyleneimines, polymers containing vinylpyridine or vinylpyridinium units, condensates of polyamines and of epichlorohydrin, quaternary polyureylenes and chitin derivatives.

The amphoteric polymers which can be used in accordance with the present invention may be chosen from the polymers containing K and M units distributed statistically in the polymer chain where K denotes a unit which is derived from a monomer containing at least one basic nitrogen atom and M denotes a unit which is derived from an acidic monomer containing one or more carboxylic or sulphonic groups or alternatively K and M may denote groups which are derived from zwitterionic monomers of carboxybetaines or of sulphobetaines;
K and M may also denote a cationic polymer chain containing primary, secondary, tertiary or quaternary amine groups, in which at least one of the amine groups carries a carboxylic or sulphonic group linked via a hydrocarbon radical or alternatively K and M form part of a chain of a polymer with an α,β-dicarboxylic ethylene unit in which one of the carboxylic groups has been caused to react with a polyamine containing one or more primary or secondary amine groups.

The amphoteric polymers corresponding to the definition given above which are more particularly preferred are chosen from the following polymers:
(1) The polymers resulting from the copolymerization of a monomer derived from a vinyl compound carrying a carboxylic group such as more particularly acrylic acid, methacrylic acid, maleic acid, alpha-chloroacrylic acid, and of a basic monomer derived from a substituted vinyl compound containing at least one basic atom such as more particularly dialkylaminoalkyl methacrylate and acrylate, dialkylaminoalkylmethacrylamide and acrylamide. Such compounds are described in American Patent No. 3,836,537. Mention may also be made of the sodium acrylate/acrylamidopropyltrimethylammonium chloride copolymer.
   The vinyl compound may also be a dialkyldiallylammonium salt such as dimethyldiallylammonium chloride.
(2) The polymers containing units which are derived from:
   a) at least one monomer chosen from acrylamides or methacrylamides substituted on the nitrogen by an alkyl radical,
   b) at least one acidic comonomer containing one or more reactive carboxylic groups, and
   c) at least one basic comonomer such as esters with primary, secondary, tertiary and quaternary amine substituents of acrylic and methacrylic acids and the product of quaternization of dimethylaminoethyl methacrylate with dimethyl or diethyl sulphate.
      The N-substituted acrylamides or methacrylamides more particularly preferred according to the invention are groups whose alkyl radicals contain from 2 to 12 carbon atoms and more particularly N-ethylacrylamide, N-tert-butylacrylamide, N-tert-octylacrylamide, N-octylacrylamide, N-decylacrylamide, N-dodecylacrylamide as well as the corresponding methacrylamides.
      The acidic comonomers are chosen more particularly from acrylic, methacrylic, crotonic, itaconic, maleic and fumaric acids as well as the alkyl monoesters having 1 to 4 carbon atoms of maleic or fumaric anhydrides or acids.
      The basic comonomers preferred are methacrylates of aminoethyl, butylaminoethyl, N,N'-dimethylaminoethyl, N-tert-butylamino ethyl.
(3) The partially or completely alkylated and crosslinked polyaminoamides derived from polyaminoamides of general formula: 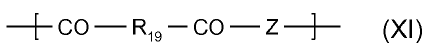 in which R₁₉ represents a divalent radical derived from a saturated dicarboxylic acid, a mono- or dicarboxylic aliphatic acid with ethylenic double bond, an ester of a lower alkanol having 1 to 6 carbon atoms of these acids or a radical which is derived from the addition of any one of the said acids with a bis-primary or bis-secondary amine, and Z denotes a radical of a bis-primary, mono- or bis-secondary polyalkylene-polyamine and preferably represents:
   a) in the proportions of 60 to 100 mol%, the radical 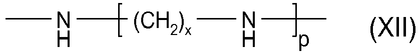 where x=2 and p=2 or 3, or alternatively x=3 and p=2
      this radical being derived from the diethylenetriamine, triethylenetetraamine or dipropylenetriamine;
   b) in the proportions of 0 to 40 mol%, the radical (XII) above, in which x=2 and p=1 and which is derived from ethylenediamine, or the radical which is derived from piperazine: 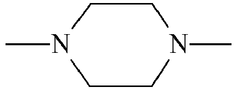
   c) in the proportions of 0 to 20 mol%, the radical -NH-(CH₂)₆-NH- which is derived from hexamethylenediamine, these polyaminoamines being crosslinked by adding a bifunctional crosslinking agent chosen from the epihalohydrins, diepoxides, dianhydrides, bis-unsaturated derivatives, by means of 0.025 to 0.35 mol of crosslinking agent per amine group of the polyaminoamide and alkylated by the action of acrylic acid, chloroacetic acid or of an alkanesultone or of their salts.
      The saturated carboxylic acids are preferably chosen from the acids having 6 to 10 carbon atoms such as adipic, 2,2,4-trimethyladipic and 2,4,4-trimethyladipic acid, terephthalic acid, the acids with an ethylene double bond such as for example acrylic, methacrylic and itaconic acids.
      The alkanesultones used in the alkylation are preferably propane or butanesultone, the salts of the alkylating agents are preferably the sodium or potassium salts.
(4) The polymers containing zwitterionic units of formula: 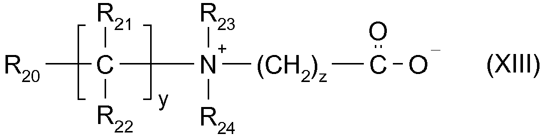 in which R₂₀ denotes a polymerizable unsaturated group such as an acrylate, methacrylate, acrylamide or methacrylamide group, y and z represent an integer from 1 to 3, R₂₁ and R₂₂ represent a hydrogen atom, methyl, ethyl or propyl, R₂₃ and R₂₄ represent a hydrogen atom or an alkyl radical such that the sum of the carbon atoms in R₂₃ and R₂₄ does not exceed 10.
   The polymers comprising such units may also comprise units derived from nonzwitterionic monomers such as dimethyl or diethylaminoethyl acrylate or methacrylate or alkyl acrylates or methacrylates, acrylamides or methacrylamides or vinyl acetate.
   By way of example, there may be mentioned the copolymer of butyl methacrylate/dimethylcarboxymethylammonioethyl methacrylate such as the product sold under the name DIAFORMER Z301^{®} by the company SANDOZ.
(7) The polymers corresponding to the general formula (XVII) as described for example in French Patent 1 400 366: 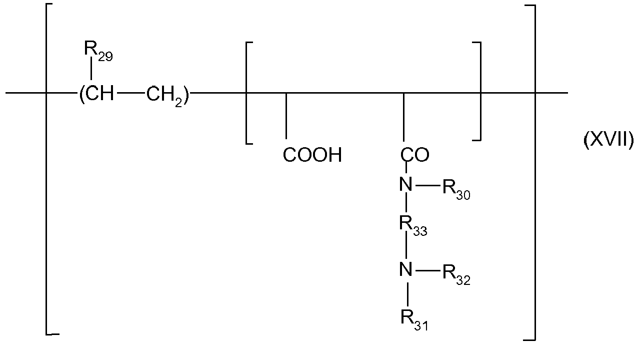
   in which R₂₉ represents a hydrogen atom, a CH₃O, CH₃CH₂O or phenyl radical, R₃₀ denotes hydrogen or a lower alkyl radical such as methyl or ethyl, R₃₁ denotes hydrogen or a lower alkyl radical such as methyl or ethyl, R₃₂ denotes a lower alkyl radical such as methyl or ethyl or a radical corresponding to the formula: -R₃₃-N(R₃₁)₂, R₃₃ representing a group -CH₂-CH₂-, -CH₂-CH₂-CH₂- or -CH₂-CH(CH₃)-, R₃₁ having the meanings mentioned above,
   as well as the higher homologues of these radicals and containing up to 6 carbon atoms,
   r is such that the molecular weight is from 500 to 6 000 000 and preferably from 1000 to 1 000 000.
(8) Amphoteric polymers of the -D-X-D-X- type chosen from:
   a) the polymers obtained by the action of chloroacetic acid or sodium chloroacetate on the compounds containing at least one unit of formula:

      -D-X-D-X-D- (XVIII)

      where D denotes a radical 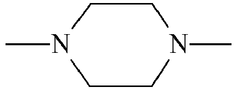 and X denotes the symbol E or E', E or E', which are identical or different, denote a bivalent radical which is an alkylene radical with a linear or branched chain containing up to 7 carbon atoms in the principal chain which is unsubstituted or substituted with hydroxyl groups and which may contain, in addition, oxygen, nitrogen or sulphur atoms, 1 to 3 aromatic and/or heterocyclic rings; the oxygen, nitrogen and sulphur atoms being present in the form of ether, thioether, sulphoxide, sulphone, sulphonium, alkylamine or alkenylamine groups, or hydroxyl, benzylamine, amine oxide, quaternary ammonium, amide, imide, alcohol, ester and/or urethane groups;
   b) the polymers of formula:

      -D-X-D-X- (XIX)

      where D denotes a radical 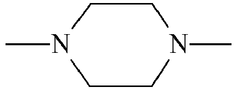 and X denotes the symbol E or E' and, at least once, E'; E having the meaning indicated above and E' is a bivalent radical which is an alkylene radical with a linear or branched chain having up to 7 carbon atoms in the principal chain, which is unsubstituted or substituted with one or more hydroxyl radicals and containing one or more nitrogen atoms, the nitrogen atom being substituted with an alkyl chain optionally interrupted by an oxygen atom and necessarily containing one or more carboxyl functional groups or one or more hydroxyl functional groups and betainized by reaction with chloroacetic acid or sodium chloroacetate.
(9) The copolymers (C₁-C₅)alkyl vinyl ether/maleic anhydride partially modified by semiamidation with an N,N-dialkylaminoalkylamine such as N,N-dimethylaminopropylamine or by semiesterification with an N,N-dialkanolamine. These copolymers may also contain other vinyl comonomers such as vinylcaprolactam.

Among all the cationic or amphoteric polymers which can be used according to the present invention, the following are preferred in particular:
(i) among the cationic polymers:
   - the homopolymer of dimethyldiallylammonium chloride such as the product sold under the name MERQUAT 100DRY by the company MERCK;
   - the copolymers of dimethyldiallylammonium chloride and acrylamide such as the products sold under the name MERQUAT 2200 by the company CALGON;
   - the polymers of the poly(quaternary ammonium) type prepared and described in French patent 2 270 846, consisting of repeating units of the following formulae (W) and (U): 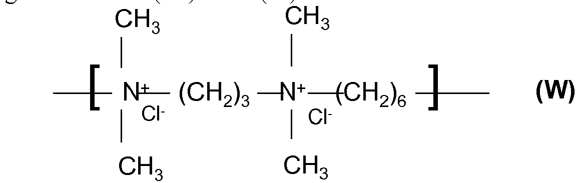 and in particular those whose molecular weight, determined by gel permeation chromatography, is between 9500 and 9900; 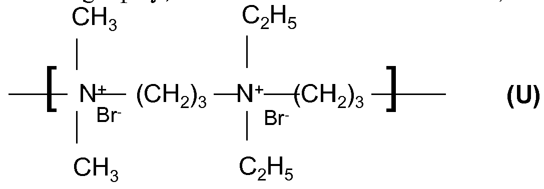 and in particular those whose molecular weight, determined by gel permeation chromatography, is about 1200;
   - the polymers of the poly(quaternary ammonium) type of the family (11) and of the formula (X) in which X⁻ denotes chlorine, and in particular those whose weight-average molecular mass is less than 100 000, preferably less than or equal to 50 000;
(ii) among the amphoteric polymers:
   - the dimethyldiallylammonium chloride/acrylic acid (80/20) copolymer marketed under the name MERQUAT 280 DRY by the company CALGON (CTFA name: POLYQUATERNIUM 22);
   - the dimethyldiallylammonium chloride/acrylic acid (95/5) copolymer marketed under the name MERQUAT 295 DRY by the company CALGON (CTFA name: POLYQUATERNIUM 22);
   - the copolymer of methacrylamidopropyltrimonium chloride, acrylic acid and ethyl acrylate marketed under the name MERQUAT 2001 by the company CALGON (CTFA name : POLYQUATERNIUM 47); and
   - the acrylamide/dimethyldiallylammonium chloride/acrylic acid terpolymer marketed under the name MERQUAT PLUS 3330 DRY by the company CALGON (CTFA name: POLYQUATERNIUM 39).

When they are present in the compositions according to the invention, the cationic and/or amphoteric polymers are present in a total proportion by weight less than or equal to 20% relative to the total weight of the composition, and preferably less than or equal to 8%.

The expression cosmetically acceptable medium is understood to mean, for the purposes of the present invention, a medium compatible with the keratin fibres and in particular the hair.

The appropriate medium for the compositions of the invention is a cosmetically acceptable medium generally comprising water or a mixture of water and at least one organic solvent. As organic solvent, there may be mentioned for example lower C₁-C₄ alkanols, such as ethanol and isopropanol; polyols and polyol ethers such as 2-butoxyethanol, propylene glycol, glycerol, propylene glycol monomethyl ether, diethylene glycol monoethyl ether and monomethyl ether, and aromatic alcohols such as benzyl alcohol or phenoxyethanol, and mixtures thereof.

The solvents are preferably present in proportions of between about 1 and 40% by weight relative to the total weight of the cosmetic composition, and more preferably still between about 5 and 30% by weight.

Of course, persons skilled in the art will be careful to choose this or these optional additional compounds such that the advantageous properties intrinsically attached to the composition in accordance with the invention are not, or not substantially, impaired by the additions envisaged.

The above adjuvants are generally present for each of them in a quantity between 0 and 20% by weight relative to the total weight of the composition.

The composition according to the invention may be provided in various forms, such as in the form of liquids, creams, gels or in any other form appropriate for dyeing keratin fibres, and in particular human hair.

Preferably, the composition according to the invention is free of nonvolatile organic amines, that is to say organic amines whose vapour pressure at 20°C is less than 0.1 mmHg. In particular, the composition of the invention is free of alkanolamine such as monoethanolamine.

The composition according to the invention may additionally comprise one or more direct dyes.

By way of example, the oxidation dye precursors are chosen from para-phenylenediamines, bisphenylalkylenediamines, para-aminophenols, ortho-aminophenols and heterocyclic bases and their addition salts with an acid or with an alkaline agent.

Among the para-phenylenediamines, there may be mentioned, by way of example, para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-p ara-p henylenediamine, N,N-diethyl-para-phenylenediamine, N,N-dipropyl-para-phenylenediamine, 4-amino-N,N-diethyl-3-methylaniline, N,N-bis(3-hydroxyethyl)-para-phenylenediamine, 4-N,N-bis(β-hydroxyethyl)amino-2-methyl aniline, 4-N,N-bis(-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylene-diamine, 2-β-hydroxyethyloxy-para-phenylenediamine, 2-β-acetylaminoethyloxy-para-phenylenediamine, N-(β-methoxyethyl)-para-phenylenediamine, and 4'-aminophenyl-1-(3-hydroxy)pyrrolidine and their addition salts with an acid or with an alkaline agent.

Among the para-phenylenediamines mentioned above, there are particularly preferred para-phenylenediamine, para-tolylenediamine, 2-isopropyl-para-phenylenediamine, 2-β-hydroxyethyl-para-phenylene-diamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine, 2-β-acetyl-aminoethyloxy-para-phenylenediamine, and their addition salts with an acid or with an alkaline agent.

Among the bisphenylalkylenediamines, there may more particularly be mentioned, by way of example, N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis (ethyl)-N,N'-bis(4'-amino-3'-methylphenyl)ethylenediamine, 1,8-bits(2,5-diaminophenoxy)-3,5-dioxaoctane, and their addition salts with an acid or with an alkaline agent.

Among the para-aminophenols, there may more particularly be mentioned, by way of example, para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, 4-amino-2-fluorophenol, and their addition salts with an acid or with an alkaline agent.

Among the ortho-aminophenols, there may more particularly be mentioned, by way of example, 2-aminophenol, 2-amino-5-methylphenol, 2-amino-6-methylphenol, 5-acetamido-2-aminophenol, and their addition salts with an acid or with an alkaline agent.

Among the heterocyclic bases, there may more particualrly be mentioned, by way of example, pyridine derivatives, pyrimidine derivatives and pyrazole derivatives and their addition salts with an acid or with an alkaline agent. There will be mentioned most particularly 1-β-hydroxyethyl-4,5-diaminopyrazole and its salts. The oxidation dye precursor(s) preferably represent from 0.005 to 15% by weight, and more preferably still from 0.01 to 10% by weight, relative to the total weight of the composition.

If the composition according to the invention contains one or more oxidation dye precursors, the the said composition preferably contains one or more couplers conventionally used for dyeing keratin fibres. Among these couplers, there may be mentioned in particular meta-phenylenediamines, meta-aminophenols, meta-diphenols and heterocyclic couplers and their addition salts with an acid or with an alkaline agent.

These couplers are more particularly chosen from 2-methyl-5-aminophenol, 5-N-(β-hydroxyethyl)amino-2-methylphenol, 3-amino-phenol, 1,3-dihydroxybenzene, 1,3-dihydroxy-2-methylbenzene, 4-chloro-1,3-dihydroxybenzene, 2,4-diamino-1-(β-hydroxyethyloxy)-benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 2,6-dihydroxy-4-methylpyridine, 1-H-3-methyl-pyrazol-5-one, 1-phenyl-3-methylpyrazol-5-one, 2,6-dimethylpyrazolo-[1,5-b]-1,2,4-triazole, 2,6-dimethyl[3,2-c]-1,2,4-triazole, 6-methyl-pyrazolo[1,5-a]-benzimidazole, and their addition salts with an acid or with an alkaline agent.

When they are present, the coupler(s) preferably represent from 0.001 to 15% by weight, and more preferably still from 0.05 to 10% by weight relative to the total weight of the composition.

In general, the addition salts of the oxidation bases and of the couplers which can be used in the context of the invention are in particular chosen from the addition salts with an acid such as the hydrochlorides, hydrobromides, sulphates, citrates, succinates, tartrates, tosylates, benzenesulphonates, phosphates and acetates and the addition salts with a base such as sodium hydroxide, potassium hydroxide, aqueous ammonia, amines and alkanolamines.

The direct dye(s) which can be used in the dyeing composition may be chosen from neutral, acidic or cationic nitro dyes of the benzene series, neutral, acidic or cationic azo direct dyes, neutral, acidic or cationic quinone and in particular anthraquinone direct dyes, azine direct dyes, triarylmethane direct dyes, indoamine direct dyes and natural direct dyes, alone or as a mixture.

Among the benzene direct dyes which can be used according to the invention, the following compounds may be mentioned without limitation:
- 1,4-diamino-2-nitrobenzene,
- 1-amino-2 nitre-4-ß- hydroxyethylaminobenzene
- 1-amino-2 nitro-4-bis(β-hydroxyethyl)aminobenzene
- 1,4-bis(ß-hydroxyethylamino)-2-nitrobenzene
- 1-ß-hydroxyethylamino-2-nitro-4-bis(β-hydro xyethylamino)b enz ene
- 1-ß-hydroxyethylamino-2-nitro-4-aminobenzene
- 1-β-hydroxyethylamino-2-nitro-4-(ethyl)(ß-hydroxyethyl)aminobenzene
- 1-amino-3-methyl-4-β-hydroxyethylamino-6-nitrobenzene
- 1-amino-2-nitro-4-β-hydroxyethylamino-5-chlorobenzene
- 1,2-diamino-4-nitrobenzene
- 1-amino-2-β-hydroxyethylamino-5-nitrobenzene
- 1,2-bis(β-hydroxyethylamino)-4-nitrobenzene
- 1-amino-2-tris(hydroxymethyl)methylamino-5-nitrobenzene
- 1-hydroxy-2-amino-5-nitrobenzene
- 1-hydroxy-2-amino-4-nitrobenzene
- 1-hydroxy-3-nitro-4-aminobenzene
- 1-P-hydroxyethyloxy-2-β-hydroxyethylamino-5-nitrobenzene
- 1-methoxy-2-β-hydroxyethylamino-5-nitrobenzene
- 1-β-hydroxyethyloxy-3-methylamino-4-nitrobenzene
- 1-β,γ-dihydroxypropyloxy-3-methylamino-4-nitrobenzene
- 1-β-hydroxyethylamino-4-β,γ-dihydroxypropyloxy-2-nitrobenzene
- 1-β,γ-dihydroxypropylamino-4-trifluoromethyl-2-nitrobenzene
- 1-β-hydroxyethylamino-4-trifluoromethyl-2-nitrobenzene
- 1-β-hydroxyethylamino-3-methyl-2-nitrobenzene
- 1-β-aminoethylamino-5-methoxy-2-nitrobenzene
- 1-hydroxy-2-chloro-6-ethylamino-4-nitrobenzene
- 1-hydroxy-2-chloro-6-amino-4-nitrobenzene
- 1-hydroxy-6-bis(β-hydroxyethyl)amino-3-nitrobenzene
- 1-β-hydroxyethylamino-2-nitrobenzene
- 1-hydroxy-4-β-hydroxyethylamino-3-nitrobenzene.

Among the azo direct dyes which can be used according to the invention, there may be mentioned the cationic azo dyes described in patent applications WO 95/15144, WO 95/01772, EP-714954, FR 2 822 696, FR 2 825 702, FR 2 825 625, FR 2 822 698, FR 2 822 693, FR 2 822 694, FR 2 829 926, FR 2 807 652, WO 02/078660, WO 02/100834, WO 02/100369 and FR 2 844 269 whose content forms an integral part of the invention.

Among these compounds the following dyes may be most particularly mentioned:
- 1,3-dimethyl-2-[[4-(dimethylamino)phenyl]azo]-1H-imidazolium chloride,
- 1,3-dimethyl-2-[(4-aminophenyl)azo]-1H-imidazolium chloride,
- 1-methyl-4-[(methylphenylhydrazono)methyl]pyridinium methyl sulphate.

There may also be mentioned, among the azo direct dyes, the following dyes, which are described in COLOUR INDEX INTERNATIONAL 3rd edition:
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24

There may also be mentioned 1-(4'-aminodiphenylazo)-2-methyl-4bis(β-hydroxyethyl) aminobenzene and 4-hydroxy-3-(2-methoxyphenylazo)-1-naphthalenesulphonic acid.

Among the quinone direct dyes, the following dyes may be mentioned:
- Acid Violet 43
- Acid Blue 62
- Basic Blue 22
- Basic Blue 99
and the following compounds:
- 1-N-methylmorpholiniumpropylamino-4-hydroxyanthraquinone
- 1-aminopropylamino-4-methylaminoanthraquinone
- 1-aminopropylaminoanthraquinone
- 5-β-hydroxyethyl-1,4-diaminoanthraquinone
- 2-aminoethylaminoanthraquinone
- 1,4-bis(β,γ-dihydroxypropylamino)anthraquinone.

Among the azine dyes, the following compounds may be mentioned:
- Basic Blue 17
- Basic Red 2.

Among the triarylmethane dyes which can be used according to the invention, the following compounds may be mentioned:
- Basic Green 1
- Acid blue 9
- Basic Violet 3
- Basic Violet 14
- Basic Blue 7
- Acid Violet 49
- Basic Blue 26
- Acid Blue 7

Among the indoamine dyes which can be used according to the invention, the following compounds may be mentioned:
- 2-β-hydroxyethylamino-5-[bis(β-4'-hydroxyethyl)amino]-anilino-1,4-benzoquinone

Among the natural direct dyes which can be used according to the invention, there may be mentioned carminic acid, kermesic acid, purpurogallin, protocatechaldehyde, isatin, curcumin, spinulosin and apigenidin. It is also possible to use extracts or decoctions containing these natural dyes and in particular henna-based poultices or extracts.

When the composition according to the invention comprises one or more direct dyes, then the the said composition is a lightening direct dye composition.

The direct dye(s) preferably represent from 0.001 to 20% by weight approximately of the total weight of the dyeing composition and still more preferably from 0.005 to 10% by weight approximately.

The present invention also relates to a method for the oxidation dyeing of keratin fibres, in particular human keratin fibres such as the hair, consisting in applying to the the said fibres a dyeing composition as defined above comprising, as dye, one or more oxidation dye precursors, optionally combined with one or more couplers, and/or one or more direct dyes in the presence of an oxidizing agent for a period sufficient to develop the desired coloration.

After a leave-in time of 5 minutes to 1 hour, preferably of about 15 minutes to 1 hour, the keratin fibres are rinsed, washed with shampoo, rinsed again and then dried.

The oxidizing agent may be added to the dyeing composition just at the time of use, or it may be used from an oxidizing composition containing it, applied simultaneously or sequentially with the dyeing composition.

According to one embodiment of this method, the dyeing composition is obtained from the mixing of an aqueous composition comprising one or more oxidizing agents and a dyeing composition comprising one or more oxidation dye precursors and/or one or more direct dyes, one or more ammonium salts of an inorganic acid or of an organic acid having from 1 to 7 carbon atoms, aqueous ammonia and one or more amino acids.

The pH of the dyeing composition, after mixing with the oxidizing composition, is preferably between 5.5 and 10.5. It is preferably between 6 and 10, inclusive.

Another subject of the invention is a multicompartment device for dyeing keratin fibres, and in particular human keratin fibres. A first compartment contains a composition comprising one or more ammonium salts of an inorganic acid or of an organic acid having from 1 to 7 carbon atoms, aqueous ammonia and one or more basic amino acids in an amount ranging from 0.5 to 10% by weight, relative to the total weight of the cosmetic composition, and one or more oxidation dye precursors, and optionally one or more direct dyes, and a second compartment contains a composition comprising one or more oxidizing agents.

The following examples serve to illustrate the invention without being limiting.

### EXAMPLES

### 1. Examples of dyeing compositions in cream form:

| | Quantities (g%) | | | | |
|---|---|---|---|---|---|
| | A1 | A2 | A3 | A4 | A5 |
| Oleic acid | 2.85 | 2.85 | 2.85 | 2.85 | 2.85 |
| Cetylstearyl alcohol (C16/C18 50/50) | 17.1 | 17.1 | 17.1 | 17.1 | 17.1 |
| Oxyethylenated oleocetyl alcohol (30 EO) | 3.8 | 3.8 | 3.8 | 3.8 | 3.8 |
| Oleyl alcohol | 2.85 | 2.85 | 2.85 | 2.85 | 2.85 |
| Hexadimethrine chloride as aqueous solution at 60% | 2.5 | 2.5 | 2.5 | 2.5 | 2.5 |
| DTPA as aqueous solution at 40% | 1.5 | 1.5 | 1.5 | 1.5 | 1.5 |
| Ammonium thiolactate as aqueous solution at 58% | 0.4 | 0.4 | 0.4 | 0.4 | 0.4 |
| Arginine sold by the company AJINOMOTO | 1.9 | 1.9 | 6.9 | 6.9 | 1.9 |
| Lysine sold by the company DEGUSSA-HULS | 5 | 5 | / | / | 5 |
| Aqueous ammonia as aqueous solution containing 20% NH₃ | 9.5 | 8 | 6 | 9.5 | 6 |
| Ammonium chloride | 2 | / | 2 | / | / |
| Ammonium acetate | / | 3 | / | / | / |
| Ammonium bicarbonate | / | / | / | 2 | / |
| Ammonium lactate as aqueous solution at 60% | / | / | / | / | 0.5 |
| 1,4-Diaminobenzene | / | 0.51 | / | 0.3 | 1 |
| 1-Hydroxy-4-methylamino-benzene hemisulphate | / | 0.085 | / | 0.063 | 0.16 |
| 1,3-Dihydroxybenzene | / | 0.38 | / | 0.24 | 0.75 |
| 1-Hydroxy-3-aminobenzene | / | 0.11 | / | 0.08 | 0.21 |
| 1-beta-Hydroxyethyloxy-2,4-diaminobenzene dihydrochloride | / | 0.025 | / | 0.005 | 0.052 |
| 1-Methyl-2-hydroxy-4-beta-hydroxyethylaminobenzene | / | 0.25 | / | 0.097 | 0.49 |
| 1-Hydroxy-4-aminobenzene | 0.545 | 0.23 | 0.545 | 0.17 | 0.44 |
| 1-Methyl-2-hydroxy-4-aminobenzene | 0.615 | 0.05 | 0.615 | 0.057 | 0.1 |
| PERFUME | 0.4 | 0.4 | 0.4 | 0.4 | 0.4 |
| DEIONIZED WATER | Qs100 | Qs100 | Qs100 | Qs100 | Qs100 |

The dyeing compositions A1, A2, A3, A4 and A5 are mixed, at the time of use, in a bowl for 2 minutes, with an aqueous oxidizing composition containing 6% hydrogen peroxide, in an amount of 1 part by weight of dyeing composition per 1.5 parts by weight of oxidizing composition. The mixtures obtained are applied to locks of natural hair which is 90% white and left in for 30 minutes. The locks are then rinsed with water, they are washed with a standard shampoo, rinsed again with water and then dried and disentangled.

The hair was dyed in intense red-copper shades for compositions A1 and A3. Furthermore, the hair is not rough.

The hair was dyed in intense mahogany, red-mahogany shades for compositions A2, A4 and A5. Furthermore, the hair was not rough.

A substantial decrease in the aqueous ammonia odour was also noted.

### 2. Additional examples of alkaline dyeing compositions in cream form:

| | Quantities (g%) | | | | | |
|---|---|---|---|---|---|---|
| | A'1 | A'2 | A'3 | A'4 | A'5 | A'6 |
| Oleic acid | 2 | 2 | 2 | 2 | 2 | 2 |
| Mixture of oleocetyl alcohols (30 EO), oxyethylenated lauryl alcohol (12 EO), oxyethylenated decyl alcohol (5 EO), oxyethylenated decyl alcohol (3 EO) | 22.7 | 22.7 | 22.7 | 22.7 | 22.7 | 22.7 |
| Oleyl alcohol | 1.8 | 1.8 | 1.8 | 1.8 | 1.8 | 1.8 |
| Monoisopropanolamide of copra acids | 4 | 4 | 4 | 4 | 4 | 4 |
| Hydroxyethylcellulose | 0.3 | 0.3 | 0.3 | 0.3 | 0.3 | 0.3 |
| Hydroxyethyl cellulose quaternized with substituted lauryldimethylammonium epoxide (POLYQUATERNIUM-24) sold under the trade name QUATRISOFT LM 200 by the company AMERCHOL | 0.15 | 0.15 | 0.15 | 0.15 | 0.15 | 0.15 |
| Glycerol | 3 | 3 | 3 | 3 | 3 | 3 |
| Hexadimethrine chloride as aqueous solution at 60% | 1.5 | 1.5 | 1.5 | 1.5 | 1.5 | 1.5 |
| Polydimethyldiallylammonium chloride nonstabilzied in water (POLYQUATERNIUM-6) | 2 | 2 | 2 | 2 | 2 | 2 |
| Ascorbic acid | 0.25 | 0.25 | 0.25 | 0.25 | 0.25 | / |
| EDTA | 0.2 | 0.2 | 0.2 | 0.2 | 0.2 | 0.2 |
| Ammonium thiolactate as aqueous solution at 58% | 0.8 | 0.8 | 0.8 | 0.8 | 0.8 | / |
| Arginine sold by the company AJINOMOTO | 1.33 | 1.33 | 5.33 | 5.33 | 1.33 | 1.33 |
| Lysine sold by the company DEGUSSA-HULS | 5 | 5 | / | / | 5 | 5 |
| Aqueous ammonia as aqueous solution containing 20% NH₃ | 9.5 | 8 | 6 | 9.5 | 6 | 8 |
| Ammonium chloride | 2 | / | 2 | / | / | / |
| Ammonium sulphate | / | / | / | / | 1 | / |
| Ammonium acetate | / | 3 | / | / | / | 0,5 |
| Ammonium bicarbonate | / | / | / | 2 | / | / |
| Ammonium lactate as aqueous solution at 60% | / | / | / | / | 0,5 | / |
| 1,4-Diaminobenzene | / | 0.51 | / | 0.3 | 1 | / |
| 1-Hydroxy-4-methylaminobenzene hemisulphate | / | 0.085 | / | 0.063 | 0.16 | / |
| 1,3-Dihydroxybenzene | / | 0.38 | / | 0.24 | 0.75 | / |
| 1-Hydroxy-3-aminobenzene | / | 0.11 | / | 0.08 | 0.21 | / |
| 1-beta-Hydroxyethyloxy-2,4-diaminobenzene | / | 0.025 | / | 0.005 | 0.052 | / |
| 1-Methyl-2-hydroxy-4-beta-hydroxyethylaminobenzene | / | 0.25 | / | 0.097 | 0.49 | / |
| 1-Hydroxy-4-aminobenzene | 0.545 | 0.23 | 0.545 | 0.17 | 0.44 | / |
| 1-Methyl-2-hydroxy-4-aminobenzene | 0.615 | 0.05 | 0.615 | 0.057 | 0.1 | / |
| Basic Red 51 | / | / | / | / | / | 2 |
| Perfume | 0.95 | 0.95 | 0.95 | 0.95 | 0.95 | 0.95 |
| Deionized water | Qs 100 | Qs 100 | Qs 100 | Qs 100 | Qs 100 | Qs 100 |

The dyeing compositions A'1, A'2, A'3, A'4, A'5 and A'6 are mixed, at the time of use, in a plastic bowl for 2 minutes, with an aqueous oxidizing composition containing 6% hydrogen peroxide, in an amount of 1 part by weight of dyeing composition per 1.5 parts by weight of oxidizing composition. The mixtures obtained are applied to locks of natural hair which is 90% white and left in for 30 minutes. The locks are then rinsed with water, they are washed with a standard shampoo, rinsed again with water and then dried and disentangled.

The hair was dyed in red-copper shades for compositions A'1, A'3 and A'6. Furthermore, the hair was not rough.

A substantial decrease in the aqueous ammonia odour was also noted.

The hair was dyed in more or less intense mahogany, red-mahogany shades for compositions A'2, A'4 and A'5. Furthermore, the hair was not rough.

## Claims

1. Composition comprising, in a cosmetically acceptable medium:
(a) one or more ammonium salts of an inorganic acid or an organic acid having from 1 to 7 carbon atoms;
(b) aqueous ammonia, and
(c) one or more basic amino acids;
wherein the basic amino acids are present in an amount ranging from 1 and 10% by weight relative to the total weight of the composition, and
(d) one or more oxidation dye precursors.

2. Composition according to Claim 1, **characterized in that** the amino acid corresponds to the formula: 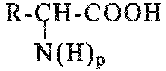 in which:
when p=2, R represents a hydrogen atom, an aliphatic group containing or not containing a heterocyclic portion, or an aromatic group,
or
when p=1, R may form with the nitrogen atom of -N(H)ₚ a heterocycle.

3. Composition according to Claim 2, **characterized in that** the aliphatic group is a linear or branched C₁₋₄ alkyl group; a linear or branched C₁₋₄ hydroxyalkyl group; a linear or branched C₁₋₄ aminoalkyl group; a linear or branched (C₁₋₄ alkyl)thio(C₁₋₄ alkyl) group; a linear or branched C₂₋₄ carboxyalkyl group; a linear or branched ureidoalkyl group, a linear or branched guanidinoalkyl group, a linear or branched imidazoloalkyl group, a linear or branched indoylalkyl group, the alkyl portions of the latter four groups containing one to four carbon atoms.

4. Composition according to Claim 2, **characterized in that** the aromatic group is a C₆ aryl or C₇₋₁₀ aralkyl group, the aromatic nucleus being optionally substituted with one or more C₁₋₄ alkyl or hydroxyl groups.

5. Composition according to Claim 2, **characterized in that** the heterocycle, when p=1, is a 5-membered saturated ring, optionally substituted with one or more C₁₋₄ alkyl groups, or hydroxyl.

6. Composition according to any one of the preceding claims, **characterized in that** the amino acid is chosen from arginine, histidine and lysine.

7. Composition according to Claim 6, **characterized in that** the amino acid is chosen from arginine and lysine.

8. Composition according to any one of the preceding claims, **characterized in that** it also comprises one or more cosmetic agents chosen from anionic, nonionic, cationic, amphoteric or zwitterionic surfactants, cationic or nonionic associative thickening polymers, vegetable oils, animal oils, mineral oils, natural or synthetic oils, fatty alcohols and mixtures thereof, cationic or amphoteric conditioning polymers.

9. Composition according to any one of the preceding claims, **characterized in that** it is free of alkanolamine.

10. Composition according to any one of the preceding claims, **characterized in that** it additionally comprises one or more direct dyes.

11. Composition according to any one of the preceding claims, **characterized in that** it additionally comprises one or more oxidizing agents.

12. Method for the oxidation dyeing of keratin fibres comprising the application to the keratin fibres of a composition as defined according to any of claims 1 to 9 and eventually comprising one or more direct dyes as defined according to Claim 10 in the presence of an oxidizing agent for a period sufficient to develop the desired coloration

13. Multicompartment device comprising a first compartment containing a dyeing composition as defined according to Claim 1, and a second compartment containing an oxidizing composition comprising one or more oxidizing agents.

## Patentansprüche

1. Zusammensetzung, die in einem kosmetisch unbedenklichen Medium
(a) ein oder mehrere Ammoniumsalze einer anorganischen Säure oder einer organischen Säure mit 1 bis 7 Kohlenstoffatomen;
(b) wässriges Ammoniak und
(c) eine oder mehrere basische Aminosäuren;
wobei die basischen Aminosäuren in einer Menge im Bereich von 1 bis 10 Gew.-%, bezogen auf das Gesamtgewicht der Zusammensetzung, vorliegen, und
(d) eine oder mehrere Oxidationsfarbstoffvorstufen umfasst.

2. Zusammensetzung nach Anspruch 1, **dadurch gekennzeichnet, dass** die Aminosäure der Formel: 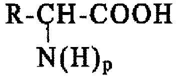 entspricht, in der:
im Fall von p = 2 R für ein Wasserstoffatom, eine aliphatische Gruppe, die gegebenenfalls einen heterocyclischen Teil enthält, oder eine aromatische Gruppe steht,
oder
im Fall von p = 1 R mit dem Stickstoffatom von -N(H)ₚ einen Heterocyclus bilden kann.

3. Zusammensetzung nach Anspruch 2, **dadurch gekennzeichnet, dass** es sich bei der aliphatischen Gruppe um eine lineare oder verzweigte C₁₋₄-Alkylgruppe; eine lineare oder verzweigte C₁₋₄-Hydroxyalkylgruppe; eine lineare oder verzweigte C₁₋₄-Aminoalkylgruppe; eine lineare oder verzweigte (C₁₋₄-Alkyl)thio(C₁₋₄-alkyl)gruppe; eine lineare oder verzweigte C₂₋₄-Carboxyalkylgruppe; eine lineare oder verzweigte Ureidoalkylgruppe, eine lineare oder verzweigte Guanidinoalkylgruppe, eine lineare oder verzweigte Imidazoloalkylgruppe oder eine lineare oder verzweigte Indolylalkylgruppe handelt, wobei die Alkylteile der letzteren vier Gruppen ein bis 4 Kohlenstoffatome enthalten.

4. Zusammensetzung nach Anspruch 2, **dadurch gekennzeichnet, dass** es sich bei der aromatischen Gruppe um eine C₆-Aryl- oder C₇₋₁₀-Aralkylgruppe handelt, wobei der aromatische Kern gegebenenfalls durch eine oder mehrere C₁₋₄-Alkyl- oder Hydroxylgruppen substituiert ist.

5. Zusammensetzung nach Anspruch 2, **dadurch gekennzeichnet, dass** es sich bei dem Heterocyclus im Fall von p = 1 um einen 5-gliedrigen gesättigten Ring, der gegebenenfalls durch eine oder mehrere C₁₋₄-Alkylgruppen oder Hydroxyl substituiert ist, handelt.

6. Zusammensetzung nach einem der vorhergehenden Ansprüche, **dadurch gekennzeichnet, dass** die Aminosäure aus Arginin, Histidin und Lysin ausgewählt ist.

7. Zusammensetzung nach Anspruch 6, **dadurch gekennzeichnet, dass** die Aminosäure aus Arginin und Lysin ausgewählt ist.

8. Zusammensetzung nach einem der vorhergehenden Ansprüche, **dadurch gekennzeichnet, dass** sie außerdem ein oder mehrere kosmetische Mittel umfasst, die aus anionischen, nichtionischen kationischen, amphoteren oder zwitterionischen Tensiden, kationischen oder nichtionischen assoziativverdickenden Polymeren, pflanzlichen Ölen, tierischen Ölen, Mineralölen, natürlichen oder synthetischen Ölen, Fettalkoholen und Mischungen davon, kationischen oder amphoteren konditionierenden Polymeren ausgewählt sind.

9. Zusammensetzung nach einem der vorhergehenden Ansprüche, **dadurch gekennzeichnet, dass** sie frei von Alkanolamin ist.

10. Zusammensetzung nach einem der vorhergehenden Ansprüche, **dadurch gekennzeichnet, dass** sie zusätzlich einen oder mehrere Direktfarbstoffe umfasst.

11. Zusammensetzung nach einem der vorhergehenden Ansprüche, **dadurch gekennzeichnet, dass** sie zusätzlich ein oder mehrere Oxidationsmittel umfasst.

12. Verfahren zum Oxidationsfärben von Keratinfasern, bei dem man auf die Keratinfasern eine Zusammensetzung gemäß einem der Ansprüche 1 bis 9, die schließlich einen oder mehrere Direktfarbstoffe gemäß Anspruch 10 umfasst, in Gegenwart eines Oxidationsmittels über einen zur Entwicklung der gewünschten Färbung ausreichenden Zeitraum aufbringt.

13. Vorrichtung mit mehreren Kompartimenten, umfassend ein erstes Kompartiment, das eine Färbezusammensetzung gemäß Anspruch 1 enthält, und ein zweites Kompartiment, das eine oxidierende Zusammensetzung, die ein oder mehrere Oxidationsmittel umfasst, enthält.

## Revendications

1. Composition comprenant, dans un milieu cosmétiquement acceptable :
(a) un ou plusieurs sels d'ammonium d'un acide inorganique ou d'un acide organique ayant de 1 à 7 atomes de carbone ;
(b) de l'ammoniaque ; et
(c) un ou plusieurs acides aminés basiques ;
où les acides aminés basiques sont présents selon une quantité allant de 1 à 10% en poids par rapport au poids total de la composition, et
(d) un ou plusieurs précurseurs de colorants d'oxydation.

2. Composition selon la revendication 1, **caractérisée en ce que** l'acide aminé correspond à la formule : 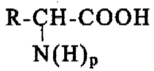 dans laquelle .
lorsque p= 2, R représente un atome d'hydrogène, un groupement aliphatique contenant ou non une portion hétérocyclique, ou un groupement aromatique, ou
lorsque p= 1, R peut former, avec l'atome d'azote de -N(H)p, un hétérocycle.

3. Composition selon la revendication 2, **caractérisée en ce que** le groupement aliphatique est un groupement C₁₋₄-alkyle linéaire ou ramifié ; un groupement C₁₋₄-hydroxyalkyle linéaire ou ramifié ; un groupement C₁₋₄-aminoalkyle linéaire ou ramifié ; un groupement (C₁₋₄-alkyl)thio(C₁₋₄-alkyl) linéaire ou ramifié ; un groupement C₂₋₄-carboxyalkyle linéaire ou ramifié ; un groupement uréidoalkyle linéaire ou ramifié ; un groupement guanidinoalkyle linéaire ou ramifié ; un groupement imiazoloalkyle linéaire ou ramifié ; un groupement indoylalkyle linéaire ou ramifié ; les portions alkyle des quatre derniers groupements contenant de un à quatre atomes de carbone.

4. Composition selon la revendication 2, **caractérisée en ce que** le groupement aromatique est un groupement C₆-aryle ou C₇₋₁₀-aralkyle, le noyau aromatique étant éventuellement substitué par un ou plusieurs groupements C₁₋₄-alkyle ou hydroxyle.

5. Composition selon la revendication 2, **caractérisée en ce que** l'hétérocycle, lorsque p=1, est un cycle saturé de 5 chaînons, éventuellement substitué par un ou plusieurs groupements C₁₋₄-alkyle, ou hydroxyle.

6. Composition selon l'une quelconque des revendications précédentes, **caractérisée en ce que** l'acide aminé est choisi parmi l'arginine, l'histidine et la lysine.

7. Composition selon la revendication 6, **caractérisée en ce que** l'acide aminé est choisi parmi l'arginine et la lysine.

8. Composition selon l'une quelconque des revendications précédentes, **caractérisée en ce qu'**elle comprend également un ou plusieurs agents cosmétiques choisis parmi des agents tensioactifs anioniques, non ioniques, cationiques, amphotères ou zwitterioniques, des polymères épaississants associatifs cationiques ou non ioniques, des huiles végétales, des huiles animales, des huiles minérales, des huiles naturelles ou synthétiques, des alcools gras et des mélanges de ceux-ci, des polymères conditionneurs cationiques ou amphotères.

9. Composition selon l'une quelconque des revendications précédentes, **caractérisée en ce qu'**elle est dépourvue d'alcanolamine.

10. Composition selon l'une quelconque des revendications précédentes, **caractérisée en ce qu'**elle comprend en outre un ou plusieurs colorants directs.

11. Composition selon l'une quelconque des revendications précédentes, **caractérisée en ce qu'**elle comprend en outre un ou plusieurs agents oxydants.

12. Procédé de coloration d'oxydation de fibres kératiniques, comprenant l'application sur lesdites fibres kératiniques d'une composition telle que définie selon l'une quelconque des revendications 1 à 9, et comprenant éventuellement un ou plusieurs colorants directs tels que définis selon la revendication 10, en présence d'un agent oxydant pendant un temps suffisant pour développer la coloration désirée.

13. Dispositif à plusieurs compartiments, comprenant un premier compartiment contenant une composition colorante telle que définie selon la revendication 1, et un deuxième compartiment contenant une composition oxydante comprenant un ou plusieurs agents oxydants.
